# EUROPEAN PATENT APPLICATION

(11) **EP 3 845 243 A1**
(43) Date of publication of application: **07.07.2021**
(21) Application number: 19812281.4
(22) Date of filing: 23.05.2019
(51) Int. Cl.: A61K 39/395

(54) **NOVEL MONOCLONAL ANTIBODY HAVING ANTI-INFLAMMATORY ACTION**

(30) Priority: 01.06.2018 JP 2018105841
(71) Applicant: National University Corporation Okayama University, Kita-ku Okayama-shi Okayama 700-8530 (JP); Sowakai Medical Foundation, Kurashiki-shi, Okayama 710-0051 (JP)
(72) Inventor: NISHIBORI, Masahiro, Okayama-shi, Okayama 700-8530 (JP); MORI, Shuji, Okayama, Okayama 7008530 (JP); MORIOKA, Yuta, Okayama, Okayama 7008530 (JP); WAKE, Hidenori, Okayama, Okayama 7008530 (JP); TOMONO, Yasuko, Okayama-shi, Okayama 701-0202 (JP)
(74) Representative: Arnold & Siedsma
(86) International application number: PCT/JP2019/020453
(87) International publication number: WO 2019/230558

(57) **Abstract**

Provided is a more effective anti-inflammatory agent having less side effects as compared to NSAIDs and steroidal anti-inflammatory agents. A therapeutic agent for inflammatory diseases containing as an active ingredient an anti-4-HNE antibody, which inhibits 4-HNE, has been found to have ameliorating effects on the expressions of various inflammation markers involved in cerebral infarction and cerebral ischemia-reperfusion, and has also been found to have life-prolonging and life-saving effects in sepsis model animals. The anti-4-HNE antibody shows remarkable efficacy even on inflammatory diseases on which existing anti-inflammatory drugs, such as NSAIDs and steroidal anti-inflammatory agents, have hitherto not shown effects.

SEQUENCE LISTING

<110>  National University Corporation Okayama University
       Sowakai Medical Foundation

<120>  A novel monoclonal antibody performing anti-inflammatory activity

<130>  GP19-1008PCT

<150>  JP P2018-105841
<151>  2018-06-01

<160>  8

<170>  PatentIn version 3.5

<210>  1
<211>  5
<212>  PRT
<213>  Rattus sp.

<400>  1

Asp Phe Tyr Met Tyr
1               5

<210>  2
<211>  19
<212>  PRT
<213>  Rattus sp.

<400>  2

Phe Ile Arg Asn Lys Ala Asn Gly Tyr Thr Thr Glu Tyr Asn Pro Ser
1               5                   10                  15

Val Lys Gly

<210>  3
<211>  12
<212>  PRT
<213>  Rattus sp.

<400>  3

Gly Tyr Tyr Gly Tyr Asn Tyr Asn Trp Phe Ala Tyr
1               5                   10

<210>  4
<211>  11
<212>  PRT
<213>  Rattus sp.

<400>  4

Arg Ala Ser Gln Ser Val Gly Ile Asn Val Asp
1               5                   10

<210>  5
<211>  7
<212>  PRT
<213>  Rattus sp.

<400>  5

Gly Ala Ser Asn Arg His Thr
1               5


<210>  6
<211>  9
<212>  PRT
<213>  Rattus sp.

<400>  6

Leu Gln Tyr Ala Ser Ile Pro Tyr Thr
1               5


<210>  7
<211>  449
<212>  PRT
<213>  Rattus sp.

<400>  7

Glu Val Lys Leu Leu Ala Ser Gly Gly Gly Leu Val Gln Pro Gly Gly
1               5                   10                  15


Ser Met Arg Leu Ser Cys Ala Ala Ser Gly Phe Thr Phe Thr Asp Phe
            20                  25                  30


Tyr Met Tyr Trp Ile Arg Gln Pro Ala Gly Lys Ala Pro Glu Trp Leu
        35                  40                  45


Gly Phe Ile Arg Asn Lys Ala Asn Gly Tyr Thr Thr Glu Tyr Asn Pro
    50                  55                  60


Ser Val Lys Gly Arg Phe Thr Ile Ser Arg Asp Asn Thr Gln Asn Met
65                  70                  75                  80


Leu Tyr Leu Gln Met Asn Thr Leu Arg Ala Glu Asp Thr Ala Thr Tyr
                85                  90                  95


Tyr Cys Ala Leu Gly Tyr Tyr Gly Tyr Asn Tyr Asn Trp Phe Ala Tyr
            100                 105                 110


Trp Gly Gln Gly Thr Leu Val Thr Val Ser Ser Ala Glu Thr Thr Ala
        115                 120                 125


Pro Ser Val Tyr Pro Leu Ala Pro Gly Thr Ala Leu Lys Ser Asn Ser

130                 135                 140

Met Val Thr Leu Gly Cys Leu Val Lys Gly Tyr Phe Pro Glu Pro Val
145                 150                 155                 160

Thr Val Thr Trp Asn Ser Gly Ala Leu Ser Ser Gly Val His Thr Phe
                165                 170                 175

Pro Ala Val Leu Gln Ser Gly Leu Tyr Thr Leu Thr Ser Ser Val Thr
            180                 185                 190

Val Pro Ser Ser Thr Trp Pro Ser Gln Thr Val Thr Cys Asn Val Ala
        195                 200                 205

His Pro Ala Ser Ser Thr Lys Val Asp Lys Lys Ile Val Pro Arg Asn
    210                 215                 220

Cys Gly Gly Asp Cys Lys Pro Cys Ile Cys Thr Gly Ser Glu Val Ser
225                 230                 235                 240

Ser Val Phe Ile Phe Pro Pro Lys Pro Lys Asp Val Leu Thr Ile Thr
                245                 250                 255

Leu Thr Pro Lys Val Thr Cys Val Val Val Asp Ile Ser Gln Asp Asp
            260                 265                 270

Pro Glu Val His Phe Ser Trp Phe Val Asp Asp Val Glu Val His Thr
        275                 280                 285

Ala Gln Thr Arg Pro Pro Glu Glu Gln Phe Asn Ser Thr Phe Arg Ser
    290                 295                 300

Val Ser Glu Leu Pro Ile Leu His Gln Asp Trp Leu Asn Gly Arg Thr
305                 310                 315                 320

Phe Arg Cys Lys Val Thr Ser Ala Ala Phe Pro Ser Pro Ile Glu Lys
                325                 330                 335

Thr Ile Ser Lys Pro Glu Gly Arg Thr Gln Val Pro His Val Tyr Thr
            340                 345                 350

Met Ser Pro Thr Lys Glu Glu Met Ile Gln Asn Glu Val Ser Ile Thr
        355                 360                 365

Cys Met Val Lys Gly Phe Tyr Pro Pro Asp Ile Tyr Val Glu Trp Gln
    370                 375                 380

Met Asn Gly Gln Pro Gln Glu Asn Tyr Lys Asn Thr Pro Pro Thr Met
385                 390                 395                 400

Asp Thr Asp Gly Ser Tyr Phe Leu Tyr Ser Lys Leu Asn Val Lys Lys
                405                 410                 415

Glu Lys Trp Gln Gln Gly Asn Thr Phe Thr Cys Ser Val Leu His Glu
            420                 425                 430

Gly Leu His Asn His His Thr Glu Lys Ser Leu Ser His Ser Pro Gly
        435                 440                 445

Lys

<210>  8
<211>  214
<212>  PRT
<213>  Rattus sp.

<400>  8

Glu Thr Val Met Thr Gln Ser Pro Thr Ser Met Ser Thr Ser Ile Gly
1               5                   10                  15

Glu Arg Val Thr Leu Asn Cys Arg Ala Ser Gln Ser Val Gly Ile Asn
            20                  25                  30

Val Asp Trp Tyr Gln Gln Thr Pro Gly Gln Ser Pro Lys Met Leu Ile
        35                  40                  45

Tyr Gly Ala Ser Asn Arg His Thr Gly Val Pro Asp Arg Phe Thr Gly
    50                  55                  60

Ser Gly Phe Gly Arg Asp Phe Thr Leu Thr Ile Ser Asn Met Glu Ala
65                  70                  75                  80

Glu Asp Leu Gly Val Tyr Tyr Cys Leu Gln Tyr Ala Ser Ile Pro Tyr
                85                  90                  95

Thr Phe Gly Ala Gly Thr Lys Leu Glu Leu Lys Arg Ala Asp Ala Ala
            100                 105                 110

Pro Thr Val Ser Ile Phe Pro Pro Ser Thr Glu Gln Leu Ala Thr Gly
        115                 120                 125

Gly Ala Ser Val Val Cys Leu Met Asn Asn Phe Tyr Pro Arg Asp Ile
    130                 135                 140

Ser Val Lys Trp Lys Ile Asp Gly Thr Glu Arg Arg Asp Gly Val Leu
145                 150                 155                 160

Asp Ser Val Thr Asp Gln Asp Ser Lys Asp Ser Thr Tyr Ser Met Ser
                165                 170                 175

Ser Thr Leu Ser Leu Thr Lys Ala Asp Tyr Glu Ser His Asn Leu Tyr
            180                 185                 190

Thr Cys Glu Val Val His Lys Thr Ser Ser Ser Pro Val Val Lys Ser
        195                 200                 205

Phe Asn Arg Asn Glu Cys
    210

## Description

### Technical Field

The present invention relates to a novel monoclonal antibody having an anti-inflammatory action.

The present application claims priority from Japanese Patent Application No. 2018-105841, which is incorporated herein by reference.

### Background Art

4-Hydroxy-2-nonenal (4-HNE) is a typical oxidative stress substance produced by oxidation of a fatty acid present in cytoplasm. It is known that 4-HNE is a cytotoxic aldehyde produced from an ω6 polyunsaturated fatty acid, such as arachidonic acid, in a biolipid, and inflicts mitochondrial damage to cause neuronal degeneration (Non Patent Literature 1: Mattson MP, Exp Gerontol, 2009, 44(10), 625-633; Non Patent Literature 2: Keller JN, et al., JNeurosci, 1998, 18(2), 687-697). In addition, 4-HNE or a structural body modified therewith has been used as a biomarker for determining a degree of oxidative stress in living bodies. However, in many cases, it has not been elucidated how 4-HNE or a 4-HNE-modified structural body of a protein acts as a molecular species serving as an extracellular signal.

Anti-inflammatory agents include steroidal anti-inflammatory drugs and non-steroidal anti-inflammatory drugs, and NSAIDs are known as the non-steroidal anti-inflammatory drugs. The NSAIDs each have an action of inhibiting cyclooxygenase (COX), which biosynthesizes prostaglandins (PGs). The NSAIDs are effective in terms of suppression of production of prostaglandins that promote inflammatory responses. However, prostaglandins have a gastrointestinal mucosa-protecting action and a renal natriuretic action, and hence the loss of these actions poses a problem as a side effect. In addition, the steroidal anti-inflammatory drugs each have a strong anti-inflammatory action. Hormones called glucocorticoids, which are secreted from the adrenal cortex, are involved in a variety of biological reactions, such as a carbohydrate metabolism-regulating action, an anti-inflammatory action, and an antiallergic action, and there are also known steroidal anti-inflammatory agents developed with a particular focus on the anti-inflammatory action out of those physiological actions. However, a corticosteroid has, in addition to the anti-inflammatory action, many side effects, such as hyperglycemia, osteoporosis, and high susceptibility to infection, and hence is used under careful patient management involving, for example, regulation of a dose.

There is a demand for development of a more effective anti-inflammatory agent having less side effects as compared to the NSAIDs and the steroidal anti-inflammatory agents.

### Citation List

### Non Patent Literature

[NPL 1] Exp Gerontol, 2009, 44(10), 625-633
[NPL 2] J Neurosci, 1998, 18(2), 687-697

### Summary of Invention

### Technical Problem

The inventors of the present invention have made extensive investigations, and as a result, have obtained a novel finding that an anti-inflammatory action can be achieved by inhibiting 4-HNE with an anti-4-HNE monoclonal antibody. The inventors of the present invention have generated a hybridoma for producing an anti-4-HNE monoclonal antibody, and have generated an anti-4-HNE monoclonal antibody of the present invention. The inventors of the present invention have recognized that the anti-4-HNE antibody of the present invention shows remarkable efficacy even on inflammatory diseases on which existing anti-inflammatory drugs, such as NSAIDs and steroidal anti-inflammatory agents, have hitherto not shown effects. Thus, the present invention has been completed.

### Solution to Problem

The prevent invention is solved by a novel anti-4-HNE monoclonal antibody. The inventors of the present invention have made extensive investigations, and as a result, have found that an anti-inflammatory action can be achieved by inhibiting 4-HNE. The inventors of the present invention have generated a hybridoma for producing an anti-4-HNE monoclonal antibody, and have generated a novel anti-4-HNE monoclonal antibody of the present invention. The inventors of the present invention have recognized that the anti-4-HNE antibody of the present invention shows remarkable efficacy even on inflammatory diseases on which existing anti-inflammatory drugs, such as NSAIDs and steroidal anti-inflammatory agents, have hitherto not shown effects. Thus, the present invention has been completed.

That is, the present invention includes the following:
1. A therapeutic agent for inflammatory diseases, including an anti-4-HNE antibody as an active ingredient.
2. The therapeutic agent for inflammatory diseases according to the above-mentioned item 1, wherein the inflammatory diseases is one kind or a plurality of kinds of diseases selected from sepsis, cerebral ischemic infarction, cerebral infarction, brain trauma, brain injury caused by brain surgery, spinal cord injury, arteriosclerosis, acute respiratory distress syndrome, acute pancreatitis, acute lung injury, lung injury caused by hemorrhagic shock, multiple organ failure, neuropathic pain, cerebral vasospasm after subarachnoid hemorrhage, burn, polytrauma, idiopathic interstitial pulmonary fibrosis, epilepsy, status epilepticus, viral encephalitis, influenza encephalopathy, inflammatory bowel disease, Kawasaki disease, multiple sclerosis, diabetic vascular complication, hepatitis, bronchial asthma, chronic bronchitis, pulmonary emphysema, organ injury after surgery, organ injury after radiotherapy, nephritis, nephrotic syndrome, acute kidney injury, hemodialysis, extracorporeal circulation, artificial respiration, acute/chronic rejection after organ transplantation, SLE, rheumatoid arthritis, DIC, autoimmune diseases, Behcet's disease, myocarditis, endocarditis, ischemia-reperfusion injury, myocardial infarction, congestive heart failure, adipose tissue inflammation, neutrophilic dermatosis, Sweet's disease, Stevens-Johnson syndrome, Reye's syndrome, cachexia, chronic fatigue syndrome, and fibromyalgia.
3. The therapeutic agent for inflammatory diseases according to the above-mentioned item 1 or 2, wherein the anti-4-HNE antibody is an antibody against a protein adduct with 4-HNE.
4. The therapeutic agent for inflammatory diseases according to any one of the above-mentioned items 1 to 3, wherein the anti-4-HNE antibody contains amino acid sequences identified by SEQ ID NOs: 1 to 6.
5. The therapeutic agent for inflammatory diseases according to any one of the above-mentioned items 1 to 3, wherein the anti-4-HNE antibody is a monoclonal antibody produced from a hybridoma identified by the accession number NITE ABP-02690.
6. A hybridoma for producing a monoclonal antibody, which is identified by the accession number NITE ABP-02690.
7. An anti-4-HNE antibody produced from a hybridoma identified by the accession number NITE ABP-02690.
8. An anti-4-HNE antibody having antigen-binding activity for 4-HNE, comprising:
   a heavy chain region containing at least one kind of amino acid sequence selected from the group consisting of amino acid sequences identified by SEQ ID NOs: 1 to 3 or the amino acid sequences each having one or a plurality of amino acids substituted, deleted, added, or inserted in the amino acid sequences identified by SEQ ID NOs: 1 to 3; and
   a light chain region containing at least one kind of amino acid sequence selected from the group consisting of the amino acid sequences identified by SEQ ID NOs: 4 to 6 or amino acid sequences each having one or a plurality of amino acids substituted, deleted, added, or inserted in the amino acid sequences identified by SEQ ID NOs: 4 to 6.
9. An anti-4-HNE antibody, including:
   a heavy chain formed of the amino acid sequence set forth in SEQ ID NO: 7; and
   a light chain formed of the amino acid sequence set forth in SEQ ID NO: 8.

A. A method of treating inflammatory diseases, including using an anti-4-HNE antibody as an active ingredient.
B. The treatment method according to the above-mentioned item A, wherein the inflammatory diseases is one kind or a plurality of kinds of diseases selected from sepsis, cerebral ischemic infarction, cerebral infarction, brain trauma, brain injury caused by brain surgery, spinal cord injury, arteriosclerosis, acute respiratory distress syndrome, acute pancreatitis, acute lung injury, lung injury caused by hemorrhagic shock, multiple organ failure, neuropathic pain, cerebral vasospasm after subarachnoid hemorrhage, burn, polytrauma, idiopathic interstitial pulmonary fibrosis, epilepsy, status epilepticus, viral encephalitis, influenza encephalopathy, inflammatory bowel disease, Kawasaki disease, multiple sclerosis, diabetic vascular complication, hepatitis, bronchial asthma, chronic bronchitis, pulmonary emphysema, organ injury after surgery, organ injury after radiotherapy, nephritis, nephrotic syndrome, acute kidney injury, hemodialysis, extracorporeal circulation, artificial respiration, acute/chronic rejection after organ transplantation, SLE, rheumatoid arthritis, DIC, autoimmune diseases, Behcet's disease, myocarditis, endocarditis, ischemia-reperfusion injury, myocardial infarction, congestive heart failure, adipose tissue inflammation, neutrophilic dermatosis, Sweet's disease, Stevens-Johnson syndrome, Reye's syndrome, cachexia, chronic fatigue syndrome, and fibromyalgia.
C. The treatment method according to the above-mentioned item A or B, wherein the anti-4-HNE antibody, which is the active ingredient, is an antibody against a protein adduct with 4-HNE.
D. The treatment method according to any one of the above-mentioned items A to C, wherein the anti-4-HNE antibody, which is the active ingredient, contains the amino acid sequences identified by SEQ ID NOs: 1 to 6.
E. The treatment method according to any one of the above-mentioned items A to C, wherein the anti-4-HNE antibody, which is the active ingredient, is a monoclonal antibody produced from a hybridoma identified by the accession number NITE ABP-02690.

### Advantageous Effects of Invention

The therapeutic agent for inflammatory diseases containing as the active ingredient the anti-4-HNE antibody of the present invention has been found to have ameliorating effects on the expressions of various inflammation markers involved in cerebral infarction and cerebral ischemia-reperfusion, and has also been found to have life-prolonging and life-saving effects in sepsis model animals. That is, it has been recognized that the anti-4-HNE antibody of the present invention shows remarkable efficacy even on inflammatory disease on which existing anti-inflammatory drugs, such as NSAIDs and steroidal anti-inflammatory agents, have hitherto not shown effects.

### Brief Description of Drawings

FIG. 1 is a diagram concerning a protein adduct for generating an anti-4-HNE antibody of the present invention, in which the modes of bonding of protein adducts each generated by allowing a protein and 4-HNE to non-enzymatically react with each other are illustrated.
FIG. 2 is a diagram for illustrating an experimental protocol involving using MCAO model rats (Experimental Example 2).
FIG. 3 includes a graph and images showing the results of an investigation of the effect of administration of the anti-4-HNE antibody on the barrier function of the blood-brain barrier (BBB) in MCAO model rats (Experimental Example 2).
FIG. 4 includes images showing the results of an investigation of a difference in cerebral infarction area through administration of the anti-4-HNE antibody in MCAO model rats (Experimental Example 2) .
FIG. 5 includes graphs showing the results of an investigation of the ameliorating effect of administration of the anti-4-HNE antibody on inflammation in MCAO model rats. Through the administration of the anti-4-HNE antibody, there were reduced the mRNA expression amounts of inflammation markers IL-1β, iNOS, and TNF-α. (Experimental Example 3)
FIG. 6 includes graphs showing the results of an investigation of the ameliorating effect of administration of the anti-4-HNE antibody on inflammation in MCAO model rats. Through the administration of the anti-4-HNE antibody, there were reduced the mRNA expression amounts of inflammation markers IL-6, IL-8R, COX2, MMP9, MMP2, and VEGF121. (Experimental Example 3)
FIGS. 7 are graphs showing the results of an investigation of the life-prolonging/life-saving effects of administration of the anti-4-HNE antibody in CLP sepsis model mice. FIG. 7A shows results as compared to PBS, and FIG. 7B shows results as compared to an anti-KLH monoclonal antibody (IgG₁ subclass) serving as a control antibody. (Experimental Example 4)
FIG. 8 is a diagram for illustrating the amino acid sequence that identifies heavy chain regions of an anti-4-HNE antibody (#13-1-1) (Experimental Example 5).
FIG. 9 is a diagram for illustrating the amino acid sequence that identifies light chain regions of the anti-4-HNE antibody (#13-1-1) (Experimental Example 5).

### Description of Embodiments

The present invention relates to a therapeutic agent for inflammatory diseases containing an anti-4-HNE antibody as an active ingredient. The present invention also encompasses a method of treating inflammatory diseases, including using an anti-4-HNE antibody as an active ingredient.

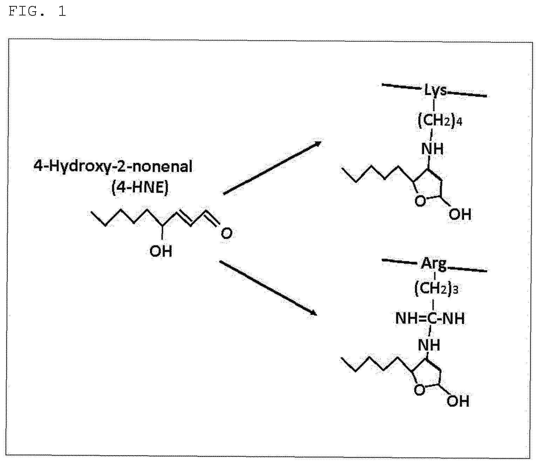

The anti-4-HNEantibody, which is the active ingredient, is an antibody against a protein adduct with 4-HNE. The anti-4-HNE antibody of the present invention can be generated using as a 4-HNE antigen a protein adduct generated by allowing a protein and 4-HNE to non-enzymatically react with each other. The present invention also relates to an anti-4-HNE antibody that is an antibody against a protein adduct with 4-HNE. The term "protein adduct with 4-HNE" refers to an adduct of 4-HNE and a protein, and refers to a product obtained through addition of two molecules, i.e., 4-HNE and a protein . Herein, the protein contained in the protein adduct is not particularly limited, but is, for example, albumin. 4-HNE reacts with primary and secondary amino groups of lysine or arginine contained in the protein, such as albumin, to form the protein adduct serving as the antigen (see FIG. 1). The present invention also relates to an anti-4-HNE antibody that is an antibody against a protein adduct with 4-HNE.

The present invention also relates to an anti-4-HNE antibody that is an antibody against a protein adduct with 4-HNE. Herein, the term "anti-4-HNE antibody" is used in its broadest sense. As long as antigen-binding activity for 4-HNE is shown, the anti-4-HNE antibody may be a monoclonal antibody, a polyclonal antibody, and/or a multispecific antibody (e.g., a bispecific antibody), or may be an antibody fragment. Examples of the antibody fragment include: Fv, Fab, Fab', Fab'-SH, or F(ab')2; a diabody; a linear antibody; and a single-chain antibody molecule (e.g., scFv). Further, the anti-4-HNE antibody of the present invention may be an antibody structure formed of a combination of various antibodies, such as the above-mentioned antibody fragments.

The class of the antibody refers to the type of constant domain or constant region included in a heavy chain (H chain) of the antibody, and examples thereof include IgA, IgD, IgE, IgG, and IgM. Herein, the class of the antibody is not particularly limited, but is most suitably IgG. As subclasses of IgG, there are given, for example, IgG₁, IgG₂, IgG₃, and IgG₄.

The anti-4-HNE antibody as used herein can be a human antibody or a humanized antibody. The human antibody refers to: an antibody produced by a human or human cells; or an antibody including the amino acid sequence corresponding to the amino acid sequences of an antibody derived from a nonhuman supply source using a human antibody repertoire or other human antibody-coding sequences . The humanized antibody can be a chimeric antibody.

The anti-4-HNE antibody of the present invention can be identified as an antibody that has a heavy chain variable region 1 (CDR H1), a heavy chain variable region 2 (CDR H2), a heavy chain variable region 3 (CDR H3), a light chain variable region 1 (CDR L1), a light chain variable region 2 (CDR L2), and a light chain variable region 3 (CDR L3), and that shows antigen-binding activity for 4-HNE. The amino acid sequences that identify the heavy chain variable regions 1 to 3 and the light chain variable regions 1 to 3 only need to be a combination of the amino acid sequences showing antigen-binding activity for 4-HNE, and are not particularly limited, but can be, for example, exemplified by the amino acid sequences set forth in SEQ ID NOs: 1 to 6. Specifically, it is suitable to contain: a heavy chain region containing at least one kind of the amino acid sequences identified by SEQ ID NOs: 1 to 3; and a light chain region containing at least one kind of the amino acid sequences identified by SEQ ID NOs: 4 to 6. Information on the amino acid sequences set forth in SEQ ID NOs: 1 to 6 is also encompassed in the scope of rights of the present invention. Besides the amino acid sequences set forth in SEQ ID NOs: 1 to 6, information on the amino acid sequences each having one or a plurality of amino acids substituted, deleted, added, or inserted therein is also encompassed in the scope of rights of the present invention as long as: the amino acid sequences each having one or a plurality of amino acids substituted, deleted, added, or inserted are included in the heavy chain or light chain of the anti-4-HNE antibody of the present invention; and antigen-binding activity for 4-HNE is shown.
·DFYMY (SEQ ID NO: 1)
·FIRNKANGYTTEYNPSVKG (SEQ ID NO: 2)
·GYYGYNYNWFAY (SEQ ID NO: 3)
·RASQSVGINVD (SEQ ID NO: 4)
·GASNRHT (SEQ ID NO: 5)
·LQYASIPYT (SEQ ID NO: 6)

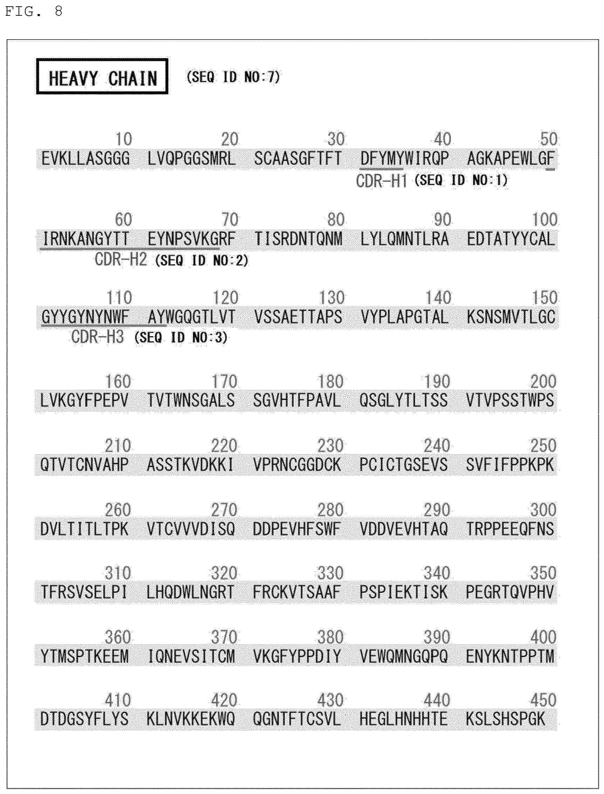

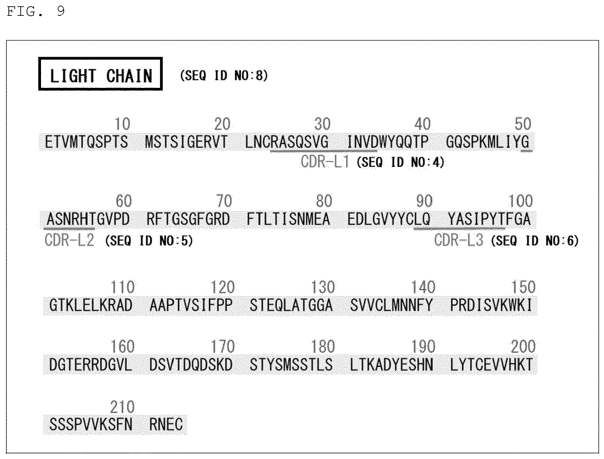

More specifically, the anti-4-HNE antibody of the present invention suitably contains the amino acid sequence forming a heavy chain illustrated in FIG. 8 (SEQ ID NO: 7), and the amino acid sequence forming a light chain illustrated in FIG. 9 (SEQ ID NO: 8) . The amino acid sequence set forth in SEQ ID NO: 7 contains the respective heavy chain variable regions identified by SEQ ID NOs: 1 to 3, and the amino acid sequence set forth in SEQ ID NO: 8 contains the respective light chain variable regions identified by SEQ ID NOs: 4 to 6. In the present invention, information on each of the amino acid sequences described above is also encompassed in the scope of rights.

As long as the anti-4-HNE antibody of the present invention shows antigen-binding activity for 4-HNE, a generation method therefor is not particularly limited. In particular, as long as the anti-4-HNE antibody of the present invention has the amino acid sequences identified above, and shows antigen-binding activity for 4-HNE, the generation method therefor is not particularly limited. The anti-4-HNE antibody can be generated, for example, from the antibody-producing hybridoma cells as described below, or can be generated, for example, by a gene recombination technique.

When the anti-4-HNE antibody of the present invention is to be generated from antibody-producing cells, the anti-4-HNE antibody can be generated by a method known per se or any method to be developed in the future with use of the above-mentioned protein adduct as the 4-HNE antigen. For example, the anti-4-HNE antibody can be generated by immunizing a mammal, such as a rat, with an antigen. When monoclonal antibody-producing hybridomas derived from a mammal, such as a rat, to be used in the present invention are to be generated through use of the 4-HNE antigen, the hybridomas can be generated in accordance with a conventional method. Anti-4-HNEmonoclonal antibody-producing hybridomas can be obtained by immunizing a rat with the 4-HNE antigen, collecting lymphocytes from the animal, and fusing myeloma cells thereto in accordance with a conventional method to obtain hybridomas.

The rat can be immunized by using as an immunogen a mixture of the 4-HNE antigen with Freund's complete adjuvant or Freund's incomplete adjuvant. A method of administering the immunogen at the time of the immunization can be any of methods known per *se,* such as subcutaneous injection, intraperitoneal injection, intravenous injection, and intramuscular injection. Of those, subcutaneous injection or intraperitoneal injection is preferred. The immunization can be performed once or a plurality of times at an appropriate interval, preferably a plurality of times at an interval of from 1 week to 5 weeks. Then, in accordance with a conventional method, monoclonal antibody-producing cells can be obtained by collecting lymph nodes from the immunized animal, subjecting lymph node cells aseptically obtained therefrom to cell fusion with myeloma cells, checking a binding property to 4-HNE by an ELISA method or the like, and repeating an operation of cloning antibody-producing hybridomas of interest. A method known per se or any method to be developed in the future can be applied as a method of generating a humanized antibody.

A specific example of the monoclonal antibody generated by the method exemplified above is a monoclonal antibody (#13-1-1) produced from a rat hybridoma (Rat hybridoma #13-1-1). The rat hybridoma was domestically deposited as the rat hybridoma (Rat hybridoma #13-1-1) to NITE Patent Microorganisms Depositary (NPMD) in room 122, 2-5-8 Kazusakamatari, Kisarazu-shi, Chiba 292-0818, Japan, on April 18, 2018. For Rat hybridoma #13-1-1, a request for transfer of microorganisms to an international deposit was accepted on May 20, 2019, and the accession number NITE ABP-02690 was assigned.

The present invention encompasses a monoclonal antibody produced from a hybridoma identified by the accession number NITE ABP-02690, and also encompasses a hybridoma for producing the monoclonal antibody.

Also when the anti-4-HNE antibody of the present invention is to be generated by a gene recombination technique, a method known per se or any method to be developed in the future can be applied. For example, the antibody can be produced by obtaining information on base sequences on the basis of the sequences of the respective variable regions of the heavy chain or the light chain identified by SEQ ID NOs: 1 to 6, and generating cDNA in accordance with a conventional method. As a method of generating the antibody by gene recombination, for example, the antibody can be generated by a method described in WO 2017/061354 A1. Besides, an anti-4-HNE antibody generated by any method other than the gene recombination technique, such as a phage display method or a synthesis method, is also encompassed in the scope of rights of the present invention as long as the method can generate the antibody based on the amino acid sequence information set forth in SEQ ID NOs: 7 and 8. Besides the amino acid sequences set forth in SEQ ID NOs: 7 and 8, the heavy chain and/or the light chain may contain the amino acid sequence forming a signal peptide.

Examples of the "inflammatory diseases" as used herein include inflammatory diseases associated with one kind or a plurality of kinds of diseases selected from sepsis, cerebral ischemic infarction, cerebral infarction, brain trauma, brain injury caused by brain surgery, spinal cord injury, arteriosclerosis, acute respiratory distress syndrome, acute pancreatitis, acute lung injury, lung injury caused by hemorrhagic shock, multiple organ failure, neuropathic pain, cerebral vasospasm after subarachnoid hemorrhage, burn, polytrauma, idiopathic interstitial pulmonary fibrosis, epilepsy, status epilepticus, viral encephalitis, influenza encephalopathy, inflammatory bowel disease, Kawasaki disease, multiple sclerosis, diabetic vascular complication, hepatitis, bronchial asthma, chronic bronchitis, pulmonary emphysema, organ injury after surgery, organ injury after radiotherapy, nephritis, nephrotic syndrome, acute kidney injury, hemodialysis, extracorporeal circulation, artificial respiration, acute/chronic rejection after organ transplantation, SLE, rheumatoid arthritis, DIC, autoimmune diseases, Behcet's disease, myocarditis, endocarditis, ischemia-reperfusion injury, myocardial infarction, congestive heart failure, adipose tissue inflammation, neutrophilic dermatosis, Sweet's disease, Stevens-Johnson syndrome, Reye's syndrome, cachexia, chronic fatigue syndrome, and fibromyalgia. In particular, the therapeutic agent effectively acts on inflammatory diseases associated with sepsis, cerebral ischemic infarction, or cerebral infarction.

The "therapeutic agent for inflammatory diseases containing an anti-4-HNE antibody as an active ingredient" of the present invention can be locally administered, or can be systemically administered. A formulation of the antibody to be used in accordance with the present invention is optionally prepared in a freeze-dried formulation or water-soluble form for storage by mixing the antibody having a desired purity with a pharmaceutically acceptable carrier, excipient, or stabilizer. Formulations for parenteral administration may include sterilized, aqueous or nonaqueous solutions, suspensions, and emulsions. Examples of nonaqueous diluents are propylene glycol, polyethylene glycol, plant oil, such as olive oil, and an organic ester composition, such as ethyl oleate, which are suitable for injection. Aqueous carriers may include water, alcoholic/aqueous solutions, emulsions, suspensions, saline, and buffered media. Parenteral carriers may include sodium chloride solution, Ringer's dextrose, dextrose and sodium chloride, lactated Ringer's, and fixed oils. Intravenous carriers may include, for example, fluid replenishers, nutrients (including monosaccharides, oligosaccharides, polysaccharides, starch, and partially degraded products of starch), and electrolytes (such as those based on Ringer's dextrose). The "therapeutic agent for inflammatory diseases containing an anti-4-HNE antibody as an active ingredient" of the present invention may further contain a preservative and other additives, such as an antimicrobial compound, an antioxidant, a chelating agent, and an inert gas.

The "therapeutic agent for inflammatory diseases containing an anti-4-HNE antibody as an active ingredient" of the present invention may contain two or more active compounds as required for a specific indication to be treated, and the compounds preferably have complementary activities that do not adversely affect each other. For example, it is sometimes desired to provide an antibody having other specificity.

The "therapeutic agent for inflammatory diseases containing an anti-4-HNE antibody as an active ingredient" of the present invention contains a therapeutically effective amount of the anti-4-HNE antibody. The "therapeutically effective amount" refers to an amount effective, at dosages and for periods of time necessary, to achieve a desired therapeutic result. The therapeutically effective amount may vary depending on factors such as the disease state, age, sex, and body weight of an individual, and the ability of the pharmaceutical to elicit a desired response in the individual.

The therapeutic agent of the present invention can be used in the following manner: a single dose or divided doses thereof are used every 24 hours, 12 hours, 8 hours, 6 hours, 4 hours, or 2 hours or any combination thereof, at least once on day 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 after the start of treatment, or at least once in week 1, 2, 3, 4, 5, 6, 7, 8, 9, 10, 11, 12, 13, 14, 15, 16, 17, 18, 19, or 20, or any combination thereof, at a daily dose in terms of daily antibody amount of from about 0.1 mg/kg to about 100 mg/kg, for example, 0.5 mg/kg, 0.9 mg/kg, 1.0 mg/kg, 1.1 mg/kg, 1.5 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg, 21 mg/kg, 22 mg/kg, 23 mg/kg, 24 mg/kg, 25 mg/kg, 26 mg/kg, 27 mg/kg, 28 mg/kg, 29 mg/kg, 30 mg/kg, 40 mg/kg, 45 mg/kg, 50 mg/kg, 60 mg/kg, 70 mg/kg, 80 mg/kg, 90 mg/kg, or 100 mg/kg.

The present invention provides the "therapeutic agent for inflammatory diseases containing an anti-4-HNE antibody as an active ingredient." A product including the therapeutic agent may include a container and a label. Suitable examples of the container include a bottle, a vial, a syringe, and a test tube. The container can be formed from any of various materials, such as a glass and a plastic. The container may hold a composition effective for treating a pathological condition, and have a sterile access port (for example, the container can be an infusion solution bag or vial having a stopper pierceable by a hypodermic injection needle) . The product may further include a second container containing a pharmaceutically acceptable buffer, such as phosphate-buffered saline, Ringer's solution, or a dextrose solution. The product may further include other materials preferred in terms of commerce and from a user's point of view, and examples thereof include other buffers, diluents, filters, needles, syringes, and package inserts with instructions for use.

### Examples

Now, the present invention is more specifically described by way of Examples and Experimental Examples . The present invention is by no means limited by the following Examples and the like, and can be carried out with appropriate modifications within a scope compatible with the spirit described above and below, all of which are encompassed in the technical scope of the present invention.

### (Example 1) Generation of Anti-4-hydroxy-2-nonenal (4-HNE) Antibody

### (1) Generation of Protein Adduct with 4-HNE

A protein adduct with 4-HNE for generating the anti-4-hydroxy-2-nonenal (4-HNE) antibody of the present invention was generated: 4-HNE (50 mM) diluted with 0.2 M phosphate buffer (pH 7.4) and 12.5 mg/mL of bovine serum albumin (BSA) dissolved in 0.2 M phosphate buffer (pH 7.4) were mixed in equal amounts. The mixed liquid was incubated at 37°C under an aseptic condition for 7 days to generate a protein adduct solution. The resultant mixed liquid was placed in a dialysis membrane and dialyzed 3 times against a dialysate fluid having a volume 100 times as large as that of the mixed liquid, to remove remaining 4-HNE. After the dialysis, the mixed liquid thus obtained was subjected to filter sterilization again, andstoredat 4 °C. The generated protein adduct solution is hereinafter referred to as 4-HNE antigen solution.

### (2) Immunization of Rat

The 1mg/mL 4-HNE antigen solution (BSA) generated in (1) above was taken in a 2 mL glass syringe, and an equal volume of Freund's complete adjuvant was taken in another 2 mL glass syringe. Those syringes were connected to each other with a connecting tube. The 4-HNE antigen solution and the adjuvant were slowly mixed through the connecting tube to afford an emulsion. A total of 0.2 mL of the resultant emulsion in 0.1 mL aliquots was administered by injection to the footpad of a hindlimb of a rat anesthetized with sevoflurane. After 2 weeks, blood was sampled from the jugular vein to recognize an increase in antibody titer, and at the same time, swollen iliac lymph nodes were aseptically removed. About 6×10⁷ cells were collected from the thus obtained two lymph nodes.

### (3) Cell Fusion and Cloning

The iliac lymph node cells and mouse myeloma SP2/O-Ag14 (SP2) cells were fused using an electrofusion method to generate fused cells (hybridomas). The resultant hybridomas were seeded in a 96-well microplate. After 1 week, 4-HNE-BSA was immobilized, and initial ELISA screening was performed. For positive wells, secondary screening was performed by a western blot method. The cells in positive wells were transferred to a 24-well microplate, and the cells were grown to a nearly confluent state of about 2×10⁵ cells/well. Then, the cells were cryopreserved in liquid nitrogen using 0.5 mL of a freezing medium (GIT medium supplemented with 10% fetal bovine serum and 10% dimethyl sulfoxide). The cryopreserved cells were thawed, and then cloned in a 96-well microplate. Hybridomas obtained through the cloning were domestically deposited as a rat hybridoma (Rat hybridoma #13-1-1) to NITE Patent Microorganisms Depositary (NPMD) in room 122, 2-5-8 Kazusakamatari, Kisarazu-shi, Chiba 292-0818, Japan, on April 18, 2018. For Rat hybridoma #13-1-1, a request for transfer of microorganisms to an international deposit was accepted on May 20, 2019, under the accession number NITE ABP-02690.

A monoclonal antibody produced from the above-mentioned rat hybridoma (Rat hybridoma #13-1-1) was adopted as the anti-4-HNE antibody (#13-1-1) of the present invention for use in the following Examples and Experimental Examples.

### (Experimental Example 1) Specificity of Anti-4-HNE Antibody

In this Experimental Example, there was investigated antigen specificity of the anti-4-HNE monoclonal antibody (#13-1-1) generated in Example 1.

In accordance with the protein adduct generation method of Example 1, 4-HNE (50 mM), glucose (D-glucose, 250 mM), glyceraldehyde (50 mM), glycolaldehyde (50 mM), methylglyoxal (50 mM), glyoxal (Methylglyoxal, 50 mM), crotonaldehyde (CRA, 50 mM), and malondialdehyde (MDA, 50 mM) diluted with 0.2 M phosphate buffer (pH 7.4) and 12.5 mg/mL of bovine (BSA), rat (Rat-SA), rabbit (Rabbit-SA), or human serumalbumin (HSA) dissolved in 0.2 Mphosphate buffer (pH 7.4) ; were mixed in equal amounts to generate respective mixed liquids. Each mixed liquid was incubated at 37°C under an aseptic condition for 3 weeks in the case of glucose, and for 7 days in the case of any other aldehyde. Each mixed liquid was placed in a dialysis membrane and dialyzed 3 times against a dialysate fluid having a volume 100 times as large as that of the mixed liquid, to remove the remaining aldehyde, to thereby prepare a mixed solution of substrate albumin and a protein-modifying substance. After the dialysis, each of the mixed liquids was subjected to filter sterilization again, and stored at 4°C.

Each mixed solution of substrate albumin and a protein-modifying substance was immobilized on a 96-wellmicroplate, and was investigated for specificity to the anti-4-HNE antibody (#13-1-1) of the present invention by an ELISA method. The results are shown in Table 1.

**Table 1**

| | | Substrate albumin | | | |
|---|---|---|---|---|---|
| | | BSA | Rat-SA | Rabbit-SA | HSA |
| | - | 0.150 | 0.088 | 0.114 | 0.118 |
| | D-glucose | 0.081 | 0.076 | 0.069 | 0.068 |
| | Glyceraldehyde | 0.070 | 0.069 | 0.069 | 0.068 |
| Protein-modifying substance | Glycolaldehyde | 0.071 | 0.070 | 0.067 | 0.071 |
| | Methylglyoxal | 0.073 | 0.074 | 0.072 | 0.069 |
| | Glyoxal | 0.066 | 0.073 | 0.073 | 0.065 |
| | CRA | 0.156 | 0.141 | 0.074 | 0.077 |
| | MDA | 0.074 | 0.080 | 0.070 | 0.065 |
| | 4-HNE | 1.107 | 1.373 | 0.663 | 0.956 |

In the table, the horizontal items represent the kind of serum albumin used, and the vertical items represent the kind of aldehyde used for generating the modified form of the protein. The anti-4-HNE antibody (#13-1-1) recognized only the modified form with 4-HNE. In addition, the anti-4-HNE antibody (#13-1-1) showed reactions with not only the mixed solution with bovine serum albumin, but also the mixed solutions with rat serum albumin, rabbit serum albumin, and human serum albumin.

### (Experimental Example 2) Amelioration of Cerebral Infarction in MCAO Model Rats

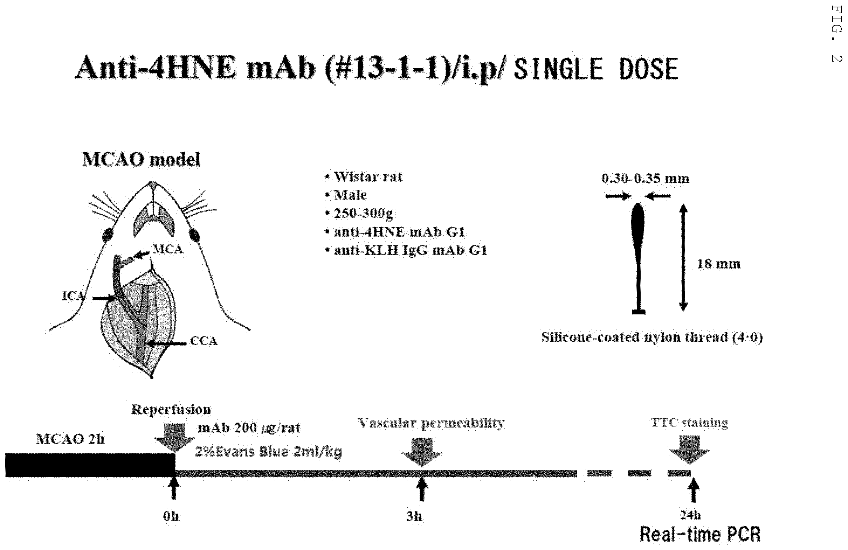

In accordance with the protocol illustrated in FIG. 2, a model (MCAO) obtained by occluding the middle cerebral artery of rats (Wister rats) for 2 hours, followed by reperfusion, was used, and immediately after the reperfusion, 1 mg/kg of the anti-4-HNE antibody (#13-1-1) and 2 mL/kg of 2% Evans blue were administered to the rats through their tail veins. As a control antibody, an anti-KLH monoclonal antibody (IgG₁ subclass) was administered in place of the anti-4-HNE antibody (#13-1-1).

### (1) Investigation of Barrier Function of Blood-Brain Barrier (BBB)

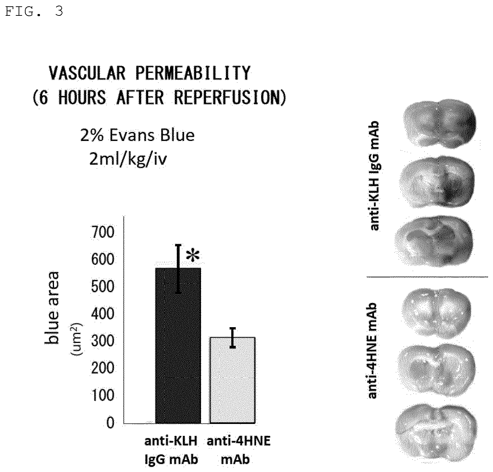

At 3 hours after the reperfusion, Evans blue was intravenously administered to the MCAO model rats. After a lapse of 6 hours from the reperfusion, systemic perfusion was performed with physiological saline. After that, the brain was excised, the brain tissue was sliced in a thickness of 2 mm, and overflowing Evans blue was quantified to investigate the barrier function of the blood-brain barrier (BBB). The area of Evans blue leakage was measured, and as a result, it was recognized that the area of Evans blue leakage was small in the case of the administration of the anti-4-HNE antibody (#13-1-1) as compared to the case of the administration of the anti-KLH monoclonal antibody (IgG₁ subclass) (FIG. 3).

### (2) Cerebral Infarction Area

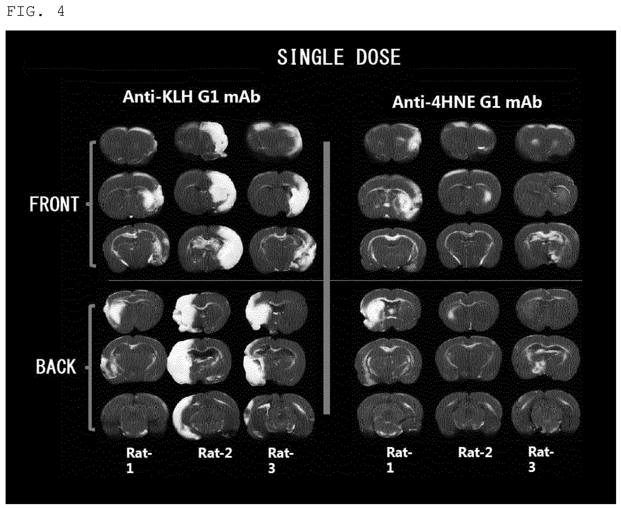

After a lapse of 24 hours from the reperfusion of the MCAO model rats, the excised brain was sliced into a coronal slice having a thickness of 2 mm and incubated at 37°C for 30 minutes while being gently shaken in a 2% solution of triphenyltetrazolium chloride (TTC) in 0.1 M phosphate buffer (pH 7.4). Then, the brain slice was fixed with a 10% formalin solution. In TTC staining, a region having mitochondrial activity develops a red color, and a non-stained region has no mitochondrial activity and can be judged to be an infarction site. A region stained white was defined as a cerebral infarction region, and its volume was measured. The results are shown in FIG. 4. As a result of the administration of the anti-4-HNE antibody (#13-1-1), it was recognized that the volume of the infarction site was reduced, and thus an ameliorating effect on cerebral infarction was recognized.

### (Experimental Example 3) Amelioration of Inflammation in MCAO Model Rats

For MCAO model rats generated by the same technique as that of Experimental Example 2, in the case of administering the anti-4-HNE antibody (#13-1-1) in the same manner as in Experimental Example 2, actions on the expressions of various inflammation-related molecules were investigated. After a lapse of 24 hours from reperfusion, 30 mg of the cerebral cortex was collected, followed by investigating changes in expression of various inflammation-related molecules.

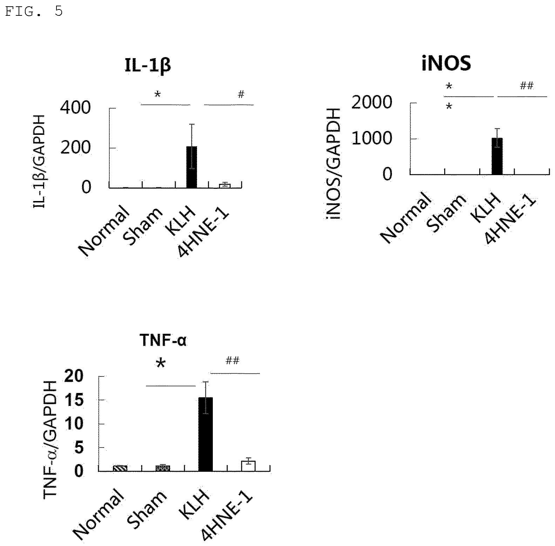

The mRNA expression amounts of inflammation-related molecules, specifically, interleukin-1β (IL-1β), inducible nitric oxide synthase (iNOS), and TNF-α were analyzed by RT-PCR. Normal, sham surgery (sham), and MCAO model (being administered the anti-KLH monoclonal antibody (IgG₁ subclass), or administered the anti-4-HNE antibody) groups were investigated, and as a result, it was recognized that the mRNA expression amounts of IL-1β, iNOS, and TNF-α were reduced in the anti-4-HNE antibody administration group to levels similar to those of the normal or sham group (FIG. 5).

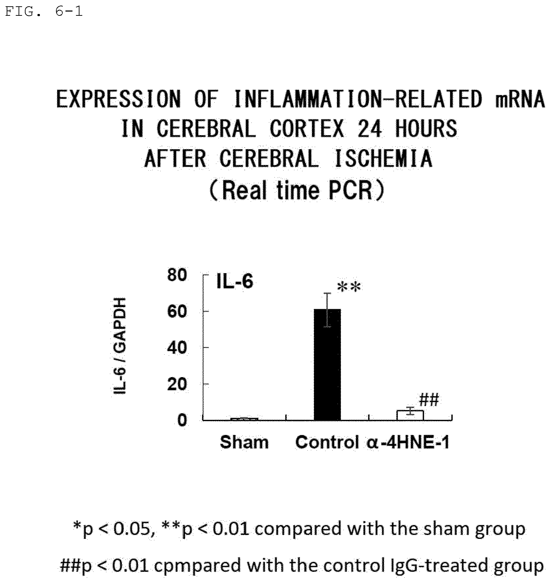

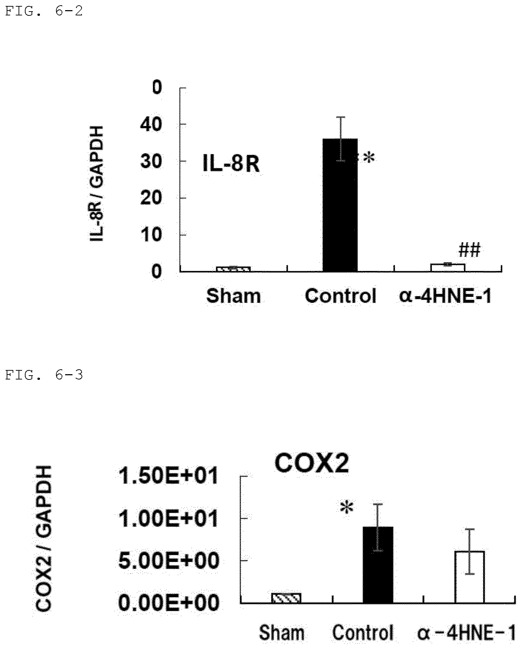

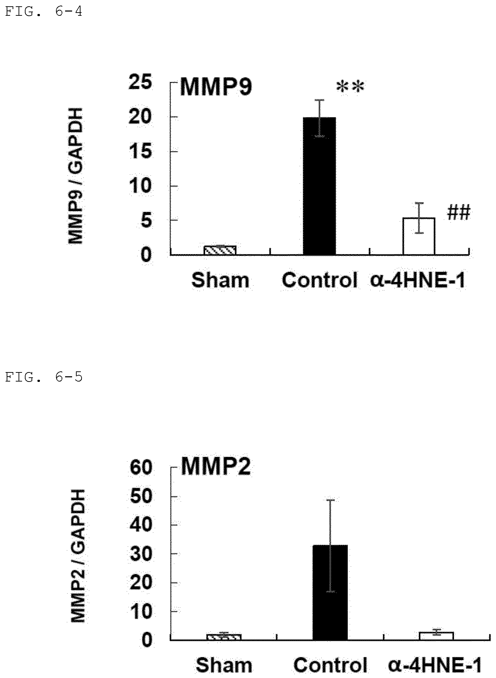

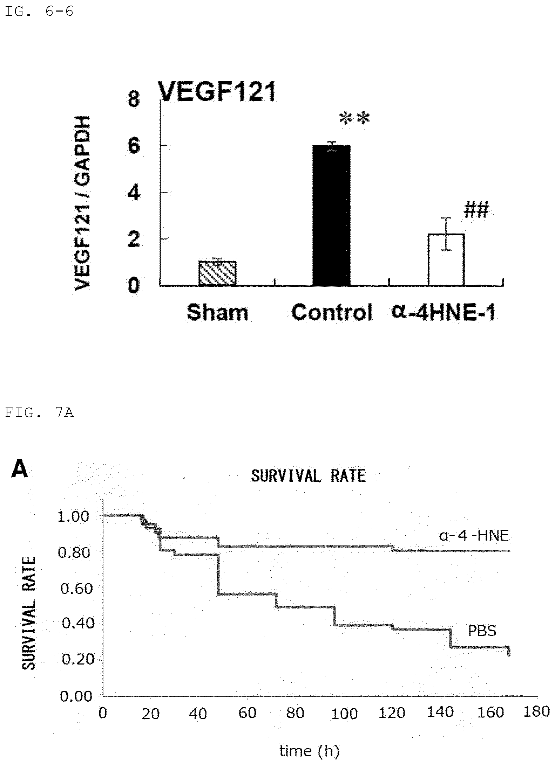

The mRNAexpression amounts of IL-6, IL-8R, COX2, matrix metalloproteinase-9 (MMP9), MMP2, and vascular endothelial growth factor (VEGF)-A121 were analyzed by RT-PCR. As a control in the MCAO model, the anti-KLH monoclonal antibody (IgG₁ subclass) was administered. As a result, it was recognized that the expressions of IL-6, IL-8R, COX2, MMP9, MMP2, and VEGF-A121 were reduced by the administration of the anti-4-HNE antibody as compared to the control (FIG. 6).

### (Experimental Example 4) Therapeutic Effect in CLP Mouse Sepsis Model

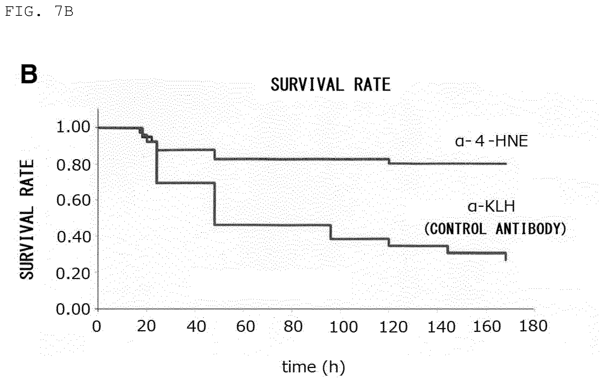

In this Experimental Example, there was investigated the therapeutic effect of the anti-4-HNE antibody (#13-1-1) in a sepsis model with cecal ligation and puncture (CLP) . The cecum was excised from the abdominal cavity of mice, the root of the cecum was ligated with a suture, and the layer of the cecal wall was punctured using an 18-gauge syringe needle to generate a CLP sepsis model. At the same time as the CLP treatment, 200 µL of phosphate buffer (Phosphate Buffered Saline: 1×PBS(-)), 1 mg/kg of the anti-4-HNE antibody (#13-1-1), or 1 mg/kg of the anti-KLH monoclonal antibody (IgG₁ subclass) serving as a control antibody was administered to the mice through their tail veins. A survival rate up to 180 hours was determined, and as a result, an evident improvement in survival rate was found in the anti-4-HNE antibody administration group as compared to the PBS or anti-KLH monoclonal antibody (IgG₁ subclass) administration group (FIG. 7A and FIG. 7B).

### (Experimental Example 5) Sequence Information Analysis of Anti-4-HNE Antibody (#13-1-1)

For the rat hybridomas (Rat hybridoma #13-1-1) generated in Example 1, RNA was extracted, and genes for the heavy chain and light chain variable region portions of the produced antibody were obtained by amplifying with RT-PCR. The sequences of the obtained fragments were analyzed to identify the structures of the antibody variable regions. Further, hypervariable regions (CDR) in the heavy chain variable regions and the light chain variable regions were identified. As a result, it was recognized that the anti-4-HNE antibody (#13-1-1) of the present invention was an antibody containing heavy chain variable regions 1 to 3 and light chain variable regions 1 to 3 identified by the following amino acid sequences.
- Heavy chain variable region 1 (CDR H1): DFYMY (SEQ ID NO: 1)
- Heavy chain variable region 2 (CDR H2): FIRNKANGYTTEYNPSVKG (SEQ ID NO: 2)
- Heavy chain variable region 3 (CDR H3): GYYGYNYNWFAY (SEQ ID NO: 3)
- Light chain variable region 1 (CDR L1): RASQSVGINVD (SEQ ID NO: 4)
- Light chain variable region 2 (CDR L2): GASNRHT (SEQ ID NO: 5)
- Light chain variable region 3 (CDR L3): LQYASIPYT (SEQ ID NO: 6)

Further, it was recognized that the heavy chain regions of the anti-4-HNE antibody (#13-1-1) of the present invention had the amino acid sequence illustrated in FIG. 8 (SEQ ID NO: 7), and the light chain regions thereof had the amino acid sequence illustrated in FIG. 9 (SEQ ID NO: 8).

### Industrial Applicability

As described in detail above, the therapeutic agent for inflammatory diseases containing as the active ingredient the anti-4-HNE antibody of the present invention has been found to have ameliorating effects on the expressions of various inflammation markers involved in cerebral infarction and cerebral ischemia-reperfusion, and has also been found to have life-prolonging and life-saving effects in sepsis model animals. That is, it has been recognized that the therapeutic agent for inflammatory diseases containing as the active ingredient the anti-4-HNE antibody of the present invention shows remarkable efficacy even on inflammatory diseases on which existing anti-inflammatory drugs, such as NSAIDs and steroidal anti-inflammatory agents, have hitherto not shown effects. It has been conceived from the above-mentioned results that the administration of the therapeutic agent for inflammatory diseases containing as the active ingredient the anti-4-HNE antibody of the present invention provides a desired therapeutic effect even in a severely ill patient, and hence has industrial utility value.

## Claims

1. A therapeutic agent for inflammatory disease(s), comprising an anti-4-HNE antibody as an active ingredient.

2. The therapeutic agent for inflammatory disease(s) according to claim 1, wherein the inflammatory disease(s) is one kind or a plurality of kinds of diseases selected from the group consisting of sepsis, cerebral ischemic infarction, cerebral infarction, brain trauma, brain injury caused by brain surgery, spinal cord injury, arteriosclerosis, acute respiratory distress syndrome, acute pancreatitis, acute lung injury, lung injury caused by hemorrhagic shock, multiple organ failure, neuropathic pain, cerebral vasospasm after subarachnoid hemorrhage, burn, polytrauma, idiopathic interstitial pulmonary fibrosis, epilepsy, status epilepticus, viral encephalitis, influenza encephalopathy, inflammatory bowel disease, Kawasaki disease, multiple sclerosis, diabetic vascular complication, hepatitis, bronchial asthma, chronic bronchitis, pulmonary emphysema, organ injury after surgery, organ injury after radiotherapy, nephritis, nephrotic syndrome, acute kidney injury, hemodialysis, extracorporeal circulation, artificial respiration, acute/chronic rejection after organ transplantation, SLE, rheumatoid arthritis, DIC, autoimmune diseases, Behcet's disease, myocarditis, endocarditis, ischemia-reperfusion injury, myocardial infarction, congestive heart failure, adipose tissue inflammation, neutrophilic dermatosis, Sweet's disease, Stevens-Johnson syndrome, Reye's syndrome, cachexia, chronic fatigue syndrome, and fibromyalgia.

3. The therapeutic agent for inflammatory disease(s) according to claim 1 or 2, wherein the anti-4-HNE antibody, which is the active ingredient, is the antibody against a protein adduct with 4-HNE.

4. The therapeutic agent for inflammatory disease (s) according to any one of claims 1 to 3, wherein the anti-4-HNE antibody comprises the amino acid sequences identified by SEQ ID NOs: 1 to 6.

5. The therapeutic agent for inflammatory disease (s) according to any one of claims 1 to 3, wherein the anti-4-HNE antibody is a monoclonal antibody produced from a hybridoma identified by the accession number NITE ABP-02690.

6. A hybridoma for producing a monoclonal antibody, which is identified by the accession number NITE ABP-02690.

7. An anti-4-HNE antibody produced from a hybridoma identified by the accession number NITE ABP-02690.

8. An anti-4-HNE antibody having antigen-binding activity for 4-HNE, comprising:
a heavy chain region comprising at least one kind of the amino acid sequence selected from the group consisting of the amino acid sequences identified by SEQ ID NOs: 1 to 3 or the amino acid sequences each comprising one or a plurality of amino acids substituted, deleted, added, or inserted in the amino acid sequences identified by SEQ ID NOS: 1 to 3; and
a light chain region comprising at least one kind of the amino acid sequence selected from the group consisting of the amino acid sequences identified by SEQ ID NOs: 4 to 6 or the amino acid sequences each comprising one or a plurality of amino acids substituted, deleted, added, or inserted in the amino acid sequences identified by SEQ ID NOs: 4 to 6.

9. An anti-4-HNE antibody, comprising:
a heavy chain formed of the amino acid sequence set forth in SEQ ID NO: 7; and
a light chain formed of the amino acid sequence set forth in SEQ ID NO: 8.
